# EUROPEAN PATENT APPLICATION

(11) **EP 4 316 842 A1**
(43) Date of publication of application: **07.02.2024**
(21) Application number: 22306180.5
(22) Date of filing: 03.08.2022
(51) Int. Cl.: B33Y 70/00, B29C 64/106, C12M 1/00, C12M 3/00, C12M 1/26

(54) **STERILE ENCLOSURE, BIOLOGICAL PRINTING DEVICE AND METHOD OF PRINTING OF A BIOLOGICAL THREE-DIMENSIONAL STRUCTURE**

(71) Applicant: SARTORIUS STEDIM FMT SAS, 13400 Aubagne (FR); UNIVERSITE CLAUDE BERNARD - LYON 1, 69100 Villeurbanne (FR); Centre National de la Recherche Scientifique (CNRS), 75016 Paris (FR); Institut National des Sciences Appliquees de Lyon (Insa Lyon), 69100 Villeurbanne (FR); Ecole Superieure de Chimie, Physique, Electronique de Lyon, 69616 Villeurbanne Cedex (FR)
(72) Inventor: Gay, Isabelle, 13124 Peypin (FR); Dufour, Alexandre, 690006 Lyon (FR); Marquette, Christophe, 69100 Villeurbanne (FR); Petiot, Emma, 69100 Villeurbanne (FR); Barbaroux, Magali, 13112 La Destrousse (FR)
(74) Representative: Derambure Conseil

(57) **Abstract**

The invention concerns a sterile enclosure (2) intended for a printing of a biological three-dimensional structure, the enclosure comprising:
- a support (4),
- a covering cap (6) comprising a rigid part (22) and a flexible part (24) extending in the prolongation of the rigid part, the covering cap (6) being sealed to the support (4) to delimit an interior chamber (32), at least one portion of the rigid part being a septum (30) adapted to be punctured by a printing needle for printing a biological three-dimensional structure in the interior chamber.

The invention also concerns a biological printing device and a method of printing.

## Description

### Field of the invention

The present invention relates to a sterile enclosure, a covering cap and a method of printing of a biological three-dimensional structure.

### Technical background

It is known to print out biological three-dimensional structures onto a printing platform of a three-dimensional printers. After the structure is formed, the printed components are immersed in a media for a cell culture process. So once printed, the biological three- dimensional structure must be transferred to a separate bioreactor for the cell culture process. However, it is difficult to maintain the sterile conditions during the transfer of the biological three-dimensional structure to the bioreactor.

To avoid this difficulty, it is also known to print out the biological three-dimensional structure in a container adapted to contain and control a culture media and which also comprises a printer or a movable robotic arm equipped with a printing head. After printing, it is no more necessary to transfer the biological three- dimensional structure in a separate bioreactor for the cell culture process.

A drawback of this operating mode is that the container is cumbersome and heavy since it must contain the printer or the movable robotic arm. As a consequence, when the biological three-dimensional structure has been formed, the printer or the movable robotic arm equipped with the printing head cannot be used to print out another structure because the container must be kept hermetically closed during the cell culture process. Due to its size and its mechanical part which are greased, it can be problematic to sterilise the printer or the movable robotic arm and to keep it sterilized. As the container is large and cumbersome, it can be hard to move it to place it in another room during the cell culture process.

### Object and summary of the invention

A first purpose of the present invention is to propose a sterile enclosure which allows an easy displacement of the printing head in at least two perpendicular directions, and in particular in three perpendicular directions without generating stress on the needle and without risk of bending or breaking of the needle. As a consequence, the shape of the biological three-dimensional structure can be better controlled. Another consequence is that the printing platform does not need to be movable along a vertical direction because the sterile enclosure allows an easy move of the printing head along this direction. Accordingly, a less complex printer can be used. In the same way, the printer can use a smaller motor because there less resistance is applied to the robotic arm.

A second purpose of the present invention is to propose a sterile enclosure that is less bulky, cumbersome and heavy. Since the printer or a part of it is not comprised in the sterile enclosure, its size and its weight are significantly reduced.

A third purpose of the present invention is to propose a sterile enclosure that can be easily moved and stored after the printing process.

A fourth purpose of the present invention is to propose a sterile enclosure which allows immediate reuse of the printer after a biological three-dimensional structure has been formed.

A fifth purpose of the present invention is to propose a sterile enclosure which simplifies both the printing of a biological three-dimensional structure and its culture in a single container.

A sixth purpose of the present invention is to propose a method of printing and culturing a three-dimensional biological structure.

These purposes have been reached by a sterile enclosure, the covering cap and a method as defined in the independent claims. Secondary features of the sterile enclosure and of the method are set out in the dependent claims.

### Brief description of figures

Fig. 1 is a perspective and schematic view of a covering cap according to a first embodiment of the present invention;
Fig. 2 is a radial section of the covering cap illustrated on figure 1;
Fig. 3 is a perspective and schematic view of a covering cap according to a second embodiment of the present invention;
Fig. 4 is a schematic view of a section of a sterile enclosure according to a first embodiment the present invention and of a source of fluid;
Fig. 5 is a schematic view of a section of a biological printing device according to the invention, of the source of fluid and of a robotic arm during a step of the printing method according to the invention;
Fig. 6 is a schematic view of a section of a biological printing device according to the invention, of the source of fluid and of a robotic arm during another step of the printing method according to the invention;
Fig. 7 is a flowchart representing the steps of a method according to the present invention;
Fig. 8 is a schematic view of a section of a sterile enclosure according to a third embodiment of the present invention and of a source of fluid.

### Detailed description of the invention

The sterile enclosure 2 according to the invention is intended to be fixed to a printing cartridge or a printing syringe for the printing of a biological three-dimensional structure in the sterile enclosure.

The printing syringe or the printing cartridge may comprise a bioink composition. A bioink composition is a biomaterial allowing to make a hydrogel comprising a cell population in a controlled three-dimensional shape. An example of bioink is described in WO 2017115056.

Preferably, for the printing operation, the printing syringe or cartridge is mounted on a displaceable printer for example on a displaceable robotic arm. The printing can also be done manually with a printing nozzle.

In the below description, the term "printing" means making a manual deposit or injection of a bioink or making a 3D-printing with a displaceable robotic arm. In the similar manner, the term "printing needle" is used to designate a needle or a deposition nozzle.

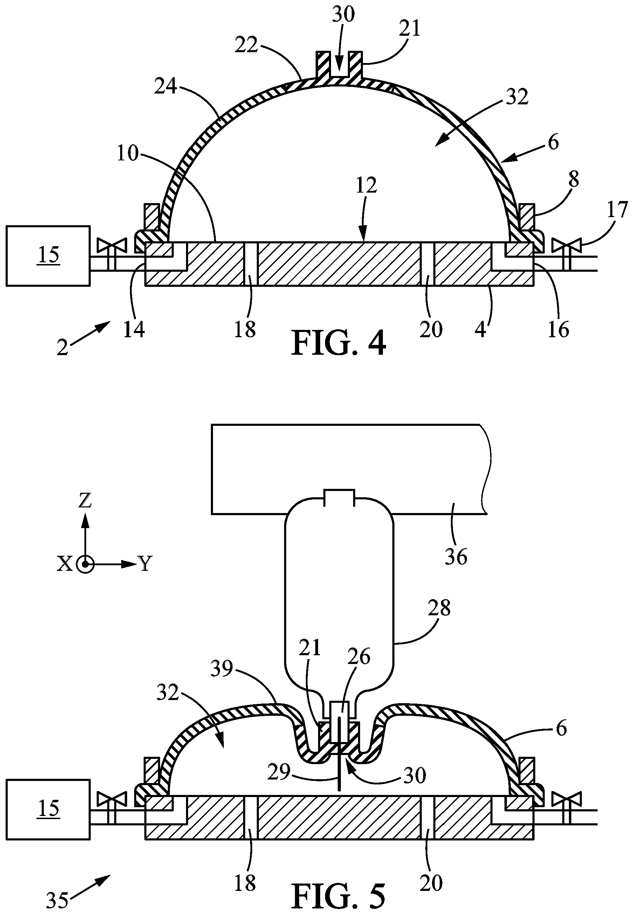

Referring to FIG. 4, the sterile enclosure 2 according to the first embodiment of the present invention comprises a support 4, a covering cap 6 and a fixation element 8 configured to fix the covering cap to the support 4 in a sealing and hermetically manner.

In the illustrated embodiment, the fixation element 8 is a clamping ring. Alternatively, the fixation element 8 can be for example hooks hinged to the support and suitable for wedging the edge of the covering cap against the edge of the upper face of the support. Alternatively, the covering cap may be sealed in another manner by welding, gluing etc.

The support 4 comprises a plate 10 having a substantially planar upper face 12 adapted to receive the bioink. The support possesses for example the shape of a disk.

The support 4 comprises at least one fluid inlet 14 for injecting fluid into the interior chamber and a fluid outlet 16 for discharging fluid from the interior chamber. In the illustrated embodiment, the fluid inlet 14 is connected to a source 15 of fluid. The fluid inlet 14 and the fluid outlet 16 is provided with a controllable valve 17.

The support 4 comprises also at least one inlet port 18 and an outlet port 20 for circulating a cell culture media. The cell culture media is used to feed the printed biological three-dimensional structure.

Alternatively, the culture media can be replaced by a solution of physiological liquid (NaCl 0.15M).

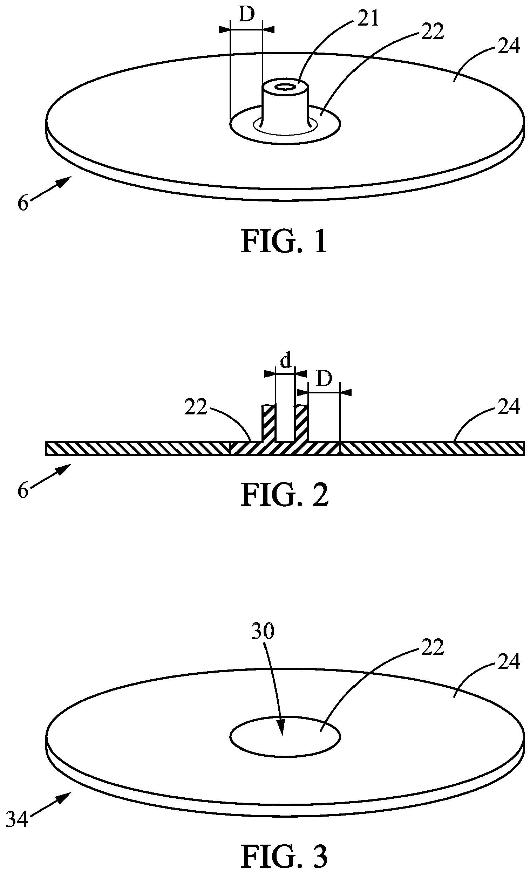

In the embodiments illustrated on figures 1 and 4, the covering cap 6 presents the general shape of a flat mat comprising a hollow cylinder 21 extending perpendicular with respect to the flat mat.

It comprises a more rigid part 22 and a more flexible part 24 surrounding the more rigid part.

To simplify the description, the most rigid part 22 will be called hereafter "rigid part" and the most flexible part will be called hereafter "flexible part". The term "flexible" means here that it bends easily without breaking.

The flexible part 24 extends in the prolongation of the rigid part. The rigid part 22 is preferably situated in the centre of the flexible part. To facilitate the comprehension of the invention, the rigid part 22 has been hatched differently.

The hollow cylinder 21 belongs to the rigid part 22 and is made with the same material as the rigid part. The hollow cylinder 21 is placed above a portion of the flat mat. The hollow cylinder is obstructed at its lower extremity by a portion of the rigid part which forms a septum.

The hollow cylinder 21 is intended to accommodate a needle adapter 26 of a printing syringe or cartridge 28 as visible on figure 5. The portion of the rigid part located inside the hollow cylinder will be punctured by a printing needle 29 of the printing syringe or cartridge. The portion of the rigid part located inside the hollow cylinder 21 forms a septum 30.

The needle adapter 26 is tightened hold by the hollow cylinder 21. Advantageously, the hollow cylinder 21 increases the stability of the printing needle 29 when the printing needle moves along two horizontal directions to print the biological three-dimensional structure.

Preferably, the rigid part 22 has a size larger than the hollow cylinder 21. In particular, the rigid part 22 extends around the hollow cylinder 21 over a radial distance D comprised between twice to five times the inside diameter d of the hollow cylinder.

Advantageously, the fact that the dimension of the rigid part 22 is greater than the diameter d of the hollow cylinder 21 contributes to avoid the tearing of the covering cap 6 under the pressure of the printing syringe and under the pressure of the fluid contained in covering cap.

Preferably, the covering cap 6 is made from an elastic material which can be easily bend, extended and expanded. For example, the covering cap 6 can be made from an elastomer.

In particular, the rigid part 22 can be made in a silicone. More preferably the rigid part 22 can be made in a liquid silicone rubber (LSR) and more preferably in liquid silicone rubber of the series 4300. For example, the LSR referenced LSR4370 can be used.

Alternatively, the rigid part 22 can be made in a thermoplastic elastomer (TPE).

The rigid part 22 has a hardness shore comprised between A40 shore and A100 shore.

The rigid part 22 has an elongation comprised between 100% to 600%. The elongation is the ratio of the extension of a material to the length of the material prior to stretching.

The flexible part 24 is made of a silicone and in particular a liquid silicone rubber (LSR) and more preferably in liquid silicone rubber of the series 4300. For example, a liquid silicone rubber referenced LSR4301 can be used.

The flexible part 24 of the covering cap is elastic and has an elongation comprised between 800% to 1100%.

The flexible part 24 of the covering cap has a hardness shore comprised between A0 shore and A30 shores, and preferably comprised between A0 shores and A15 shores.

The flexible part 24 of the covering cap has a tack comprised between to 3 millijoules per square centimeter to 9 millijoules per square centimeter, and preferably comprised between 6 to 9 millijoules per square centimeter. The tack is for example measured by using the tack-probe method described on internet at the following address : https://adhesives.specialchem.com/selection-guide/test-methods-to-evaluate-tack.

Advantageously, this tack allows to make the covering cap 6 adhere to the support 4 and to keep the interior chamber 32 hermetically closed.

The covering cap can be formed by bi-material moulding.

Alternatively, the rigid part 22 is made by 3D-printing and the flexible part 24 is formed by moulding.

Before printing, the source of fluid 15 is configured to introduce a fluid into the fluid inlet 14. Under the action of fluid pressure, the flat mat of the covering cap illustrated on figure 1 expands to form a hemisphere as shown in figure 4. The covering cap 6 and the support 4 form a casing hermetically closed. This casing delimit an interior chamber 32 wherein the biological three-dimensional structure can be printed.

The fluid introduced into the interior chamber by the source 15 can be a gas such as sterile air, carbon dioxide, oxygen, ammonia or a neutral gas such as nitrogen.

The gas is adapted to expand the volume of the interior chamber. The gas applies a pression on the internal face of the covering cap 6 to expand it.

Alternatively, the fluid introduced into the interior chamber can be a gel. In this case, the covering cap is fill with gel in order to make a suspended printing of a biological three-dimensional structure inside.

In this case, the gel is for example a gel among a Pluronic^{®} acid F-127 gel at a concentration between 15 and 30% in water or HBSS medium commercialised by Merck (registered Mark), a carbopol gel and a gelatine particle suspension. According to a second embodiment illustrated on figure 3, the covering cap 34 does not comprise a hollow cylinder 21. It presents the shape of a flat mat. According to this embodiment, the total surface of the rigid part 22 can be punctured by a printing needle. The rigid part 22 forms the septum 30.

Alternatively, the covering cap 6 has a hemispheric shape like a bell shape or a cup shape.

The support is preferably made from a medical grade material.

The support 4 can be a single use support made for example from a rigid polymer material such as a thermoplastic or thermoset.

Advantageously, this single use support can be set up very quickly anywhere at any time.

Advantageously, this support is disposable. A substantive gain of time can be made between two printings because it does no need to be washed and sterilised. No specific detergent needs to be bought and stored.

Alternatively, the support can be made from an inorganic material like for example a glass or a ceramic or in a metal such a stainless steel.

Advantageously, a support in metal can be washed and sterilised several times. The sterile enclosure can comprise a cap which is not represented and which is adapted to accommodate in the hollow cylinder 21 to close the sterile enclosure after printing.

Alternatively, the sterile enclosure comprises a clamp adapted to jam the covering cap to separate the septum from the interior chamber after printing.

Alternatively, the covering cap 6, 34,58 can be made with the following material: Unsaturated rubbers that can be cured by sulfur vulcanization:
- Natural polyisoprene: cis-1,4-polyisoprene natural rubber (NR) and trans-1,4-polyisoprene gutta-percha
- Synthetic polyisoprene (IR for isoprene rubber)
- Polybutadiene (BR for butadiene rubber)
- Chloroprene rubber (CR), polychloroprene, Neoprene, Baypren etc.
- Butyl rubber (copolymer of isobutene and isoprene, IIR)

### o Halogenated butyl rubbers (chloro butyl rubber: CIIR; bromo butyl rubber: BIIR)

- Styrene-butadiene rubber (copolymer of styrene and butadiene, SBR)
- Nitrile rubber (copolymer of butadiene and acrylonitrile, NBR), also called Buna N rubbers

### o Hydrogenated nitrile rubbers (HNBR) Therban and Zetpol (Unsaturated rubbers can also be cured by non-sulfur vulcanization if desired.)

Saturated rubbers that cannot be cured by sulfur vulcanization:
- EPM (ethylene propylene rubber, a copolymer of ethene and propene) and EPDM rubber (ethylene propylene diene rubber, a terpolymer of ethylene, propylene and a diene-component)
- Epichlorohydrin rubber (ECO)
- Polyacrylic rubber (ACM, ABR)
- Silicone rubber (SI, Q, VMQ)
- Fluorosilicone rubber (FVMQ)
- Fluoroelastomers (FKM, and FEPM) Viton, Tecnoflon, Fluorel, Aflas and Dai-El
- Perfluoroelastomers (FFKM) Tecnoflon PFR, Kalrez, Chemraz, Perlast
- Polyether block amides (PEBA)
- Chlorosulfonated polyethylene (CSM), (Hypalon)
- Ethylene-vinyl acetate (EVA)

Various other types of 4S elastomers:
- Thermoplastic elastomers (TPE)
- The proteins resilin and elastin
- Polysulfide rubber
- Elastolefin, elastic fiber used in fabric production
- Poly(dichlorophosphazene), an "inorganic rubber" from hexachlorophosphazene polymerization.

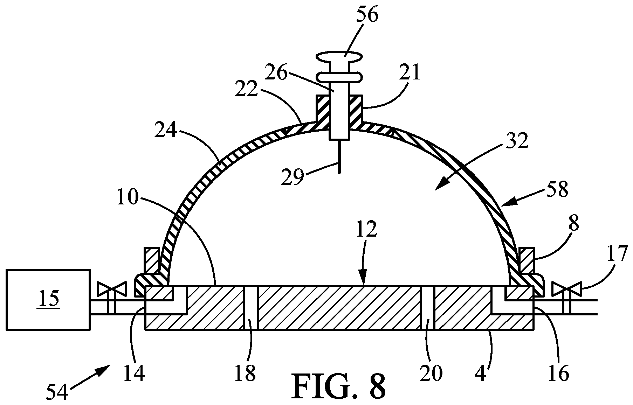

Referring to figure 8, the sterile enclosure 54 according to a third embodiment of the present invention is similar to the sterile enclosure 2 according to the first embodiment at the exception of the fact that the covering cap is different from the covering cap illustrated on figure 1 and 3. In particular, the hollow cylinder 21 of figure 1 is not obstructed by a portion of the rigid part which forms a septum. In this third embodiment, the covering cap 58 comprises a rigid part 22 and a flexible part 24 extending in the prolongation of the rigid part. The rigid part 22 comprises a through hole in the continuation of the hollow cylinder 21. A needle adapter 26 goes through the through hole of the covering cap. The needle adapter 26 is sealed to the internal face of the hollow cylinder and to the internal face of the through hole.

The needle adapter 26 is the tip that is usually connected to the open end of a barrel of a medical syringe. The needle adapter 26 comprises a needle hub holding the needle and a connector part 56 configured to be connected to a corresponding connector part of the barrel of the syringe or of a cartridge. The connector part and the corresponding connector part are for example a Luer lock connector. Advantageously, the sterile enclosure 54 illustrated on figure 8 comprises a needle protection cover adapted to be snap on an abutment ring of the needle adapter.

The other technical elements of the second embodiment identical to the technical elements of the first embodiment have been referenced by the same references and will not be described again. In particular, the covering cap, the needle adapter 26 and the support delimit a hermetically closed casing.

Figure 5 represents schematically a biological printing device 35 according to the invention. The biological printing device 35 comprises a sterile enclosure 2 as described in relation to the figures 1 to 4 a printing cartridge 28 or a printing syringe. The printing cartridge or the printing syringe 28 has a needle adapter 26 holding a printing needle 29. The printing needle 29 goes through the septum 30 of the sterile enclosure. The printing cartridge or the printing syringe 28 is fixed to a robotic arm 36 adapted to move along the three directions X, Y, Z of an orthogonal coordinate system.

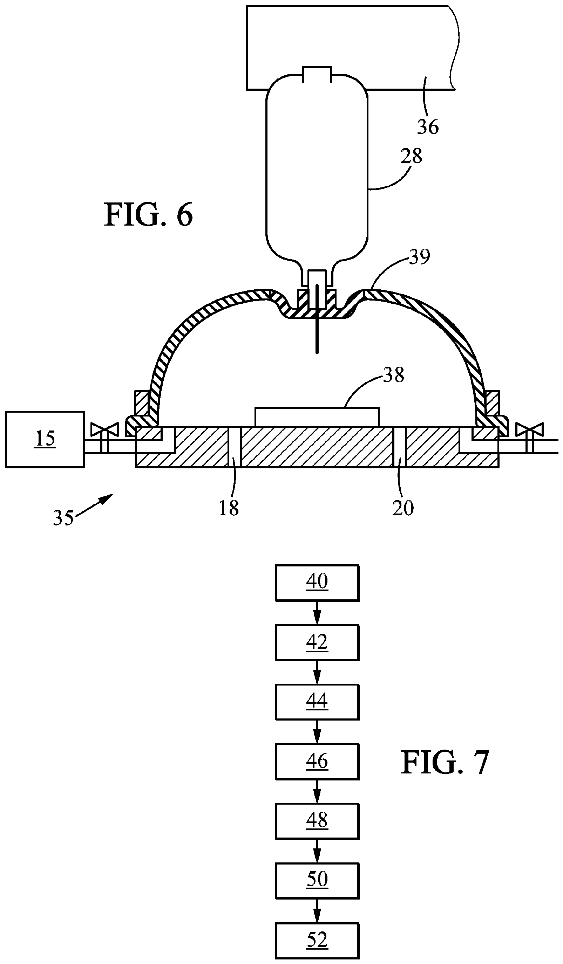

In reference to figure 7, the method of printing of a biological three-dimensional structure begins with a first injection 40 of fluid into the interior chamber 32 to inflate the covering cap 6.

Preferably, the fluid introduced into the interior chamber by the source 15 is a gas such as sterile air, carbon dioxide, oxygen ammonia or a neutral gas.

When the printing is a suspended printing, the fluid introduced into the interior chamber is a gel.

When the biological printing device illustrated on Figure 5 is used, during a step 42, the printing needle or a deposition nozzle drills the septum 30 and is introduced into the interior chamber 32. The needle adapter 26 is inserted into the hollow cylinder 21. The needle adapter 26 is held in the hollow cylinder 21.

When the sterile enclosure illustrated on figure 8 is used, during the step 42, a printing syringe or cartridge is fixed to the connector 56. The printing syringe or the printing cartridge is fixed to a robotic arm as visible on Figure 5.

During a step 44, deposition material in particular bioink is deposited into the interior chamber through the inserted needle or nozzle.

During a step 46, the robotic arm 36 moves the printing cartridge or a syringe 28 with respect to the support 4 while bioink is deposited into the interior chamber 32. During this step, a biological three-dimensional structure 36 is printed on the support as shown in figure 6 or in the gel contained in the interior chamber 32 in case of a suspended printing.

Advantageously, the pressure of the fluid on the internal face of the covering cap prevents the wall of the covering cap to come into contact with the upper face 12 of the support 4 or with the biological three-dimensional structure during the printing operation.

Advantageously, the pressure of the fluid in the interior chamber is chosen such that the tension on the covering cap is not important so that there is little pressure on the printing needle 29. So, the move of the printing needle 29 is not hindered by the covering cap which protect the biological three-dimensional structure from a non-sterile area.

Advantageously, the printing process does not need to be performed under a laminar flow hood or biological safety cabinets.

Advantageously, the volume of fluid introduced into the interior chamber 32 is adapted to the volume of the biological three-dimensional structure to be printed. During a step 48, illustrated on figure 6, fluid is injected a second times into the interior chamber 32 to expand the volume of the covering cap under the pressure of the fluid. So, the volume of the interior chamber is gradually adapted to the size of the already printed biological three-dimensional structure 38 during the printing process. Since the volume of the interior chamber can increase during the printing process, the tension on the printing needle 29 is less important. During the printing process the pressure in the interior chamber 42 is adapted such that there is a short distance between the upper part 39 of the covering cap and the septum 30. This short distance ensure that the tension generated by the presence of the covering cap on the printing needle is not important.

During a step 50, deposition material in particular bioink is deposited into the interior chamber through the inserted needle or nozzle. This deposition constitutes a second printing a biological three-dimensional structure on the already printed three-dimensional biological structure 38.

During a step 52, the fluid is evacuated, and a culture media is introduced into the interior chamber for feeding the biological three-dimensional structure.

Alternatively, the method comprises only the steps 40 to 46.

## Claims

1. Sterile enclosure (2) intended for a printing of a biological three-dimensional structure, the enclosure comprising:
- a support (4),
- a covering cap (6,34) comprising a rigid part (22) and a flexible part (24) extending in the prolongation of the rigid part, the covering cap (6,34) being sealed to the support (4) to delimit an interior chamber (32), at least one portion of the rigid part being a septum (30) adapted to be punctured by a printing needle (29) for printing a biological three-dimensional structure in the interior chamber.

2. Sterile enclosure (2) according to claim 1, wherein the covering cap (6,34) comprises at least one hollow cylinder (21) protruding from the rigid part (22), the hollow cylinder being adapted to accommodate a needle adapter (26) holding the printing needle (29), the portion of the rigid part located inside the hollow cylinder forming the septum (30).

3. Sterile enclosure (54) intended for a printing of a biological three-dimensional structure, the enclosure comprising:
- a support (4),
- a covering cap (58) sealed to the support (4) to delimit an interior chamber (32), the covering cap (58) comprising a rigid part (22) and a flexible part (24) extending in the prolongation of the rigid part, the rigid part (22) being provided with at least one hollow cylinder (21),and
- a needle adapter (26) sealed inside the hollow cylinder (21).

4. Sterile enclosure (2, 54) according to any of the claims 1 to 3, wherein the covering cap (6,34,58) is made in an elastic material, preferably an elastomer.

5. Sterile enclosure (2, 54) according to any of the claims 1 to 4, wherein the support (4) comprises at least one fluid inlet (14) for injecting fluid into the interior chamber (32), the flexible part (24) of the covering cap (6,34, 58) being adapted to expand under the pressure of the fluid contained into the interior chamber (32).

6. Sterile enclosure (2, 54) according to any of claim1 to 5, wherein the rigid part (22) of the covering cap is made in a thermoplastic elastomer (TPE) or in a silicone and in particular in a liquid silicone rubber (LSR) and more preferably in liquid silicone rubber of the serie 4300.

7. Sterile enclosure (2, 54) according to any of claim1 to 6, wherein the rigid part (22) of the covering cap has a hardness shore comprised between A40 shore and A100 shore.

8. Sterile enclosure (2, 54) according to any of claim1 to 7, wherein the rigid part (22) of the covering cap (6,34, 58) has an elongation comprised between 100% to 600%.

9. Sterile enclosure (2, 54) according to any of claims 2 to 8, wherein the rigid part (22) extends around the how cylinder (21) over a radial distance (D) comprised between twice to five times an inside diameter (d) of the hollow cylinder (21).

10. Sterile enclosure (2, 54) according to any of claim1 to 9, wherein the flexible part (24) is made of a silicone and in particular a liquid silicone rubber (LSR) and more preferably in liquid silicone rubber of the series 4300.

11. Sterile enclosure (2, 54) according to any of claim1 to 10, wherein the flexible part (24) of the covering cap (6,34,58) is elastic and has an elongation comprised between 800% to 1100%.

12. Sterile enclosure (2, 54) according to any of claim1 to 11, wherein the flexible part (24) of the covering cap (6,34,58) has a hardness shore comprised between A0 shore and A30 shore, and preferably comprised between A0 shore and A15 shore.

13. Sterile enclosure (2, 54) according to any of claim1 to 12, wherein the flexible part (24) of the covering cap (6,34,58) has a tack comprised between to 3 millijoules per square centimeter to 9 millijoules per square centimeter, and preferably comprised between 6 to 9 millijoules per square centimeter.

14. Sterile enclosure (2, 54) according to any of claim1 to 13, which comprises a clamping ring (8) configured to fix the covering cap (6,34,58) to the support (4) in a hermetic and sealed manner.

15. Sterile enclosure (2, 54) according to any of claim1 to 14, wherein the support (4) is made of one material among a polymer rigid material, a metal or an inorganic material.

16. Sterile enclosure (2, 54) according to any of claim1 to 15, wherein the covering cap (6,34, 58) is formed by bi-material moulding.

17. Sterile enclosure (2, 54) according to any of claim1 to 16, wherein the rigid part (22) is made by 3D-printing and the flexible part (24) is formed by moulding.

18. Sterile enclosure (2, 54) according to any of claim1 to 17, wherein the support (4) comprises at least one inlet port (18) and an outlet port (20) for circulating a cell culture media or a solution of physiological liquid.

19. Method of printing of a biological three-dimensional structure with a sterile enclosure (2, 54) according to any 1 to 18 and a printing cartridge (28) conform to be fixed to or having a needle adapter (26), the needle adapter (26) holding a printing needle (29), the method comprises:
- a first injection (40) of fluid into the interior chamber (32) to inflate the covering cap (6, 34) and
- a first printing (44) of a biological three-dimensional structure (38) in the interior chamber (32).

20. Method of printing according to claim 19, which further comprises the following steps:
- a second injection (48) of fluid into the interior chamber to expand the covering cap (6, 34, 58) under the pressure of the fluid,
- a second printing (50) a biological three-dimensional structure on the printed three-dimensional biological structure (38).

21. Method according to any of the claims 19 and 20, wherein the fluid injected in the interior chamber is a gel or a gas among neutral gas, sterile air, carbon dioxide, oxygen and ammonia.

22. Covering cap (6, 34) for a sterile enclosure intended for a printing of a biological three-dimensional structure, the covering cap comprising a rigid part (22) and a flexible part (24) extending in the prolongation of the rigid part, at least one portion of the rigid part forming a septum (30) adapted to be punctured by a printing needle for printing a biological three-dimensional structure.
